(19) Europäisches Patentamt
European Patent Office
Office européen des brevets

(11) EP 2 518 649 A1

(12) EUROPEAN PATENT APPLICATION

(43) Date of publication:
31.10.2012 Bulletin 2012/44

(51) Int Cl.:
$G06F\ 19/00$ $^{(2011.01)}$

(21) Application number: 11163784.9

(22) Date of filing: 26.04.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Fujitsu Limited
Kawasaki-shi, Kanagawa 211-8588 (JP)

(72) Inventors:
• Hisatomi, Makiko
Hayes, Middlesex UB4 8FE (GB)

• Kamakari, Ryota
Kawasaki-shi Kanagawa 211-8588 (JP)

(74) Representative: Wilding, Frances Ward
Haseltine Lake LLP
Lincoln House, 5th Floor
300 High Holborn
London WC1V 7JH (GB)

Remarks:
Amended claims in accordance with Rule 137(2)
EPC.

(54) **Apparatus and computer-implemented method for nutrient-based selection of ingestible products**

(57) The present invention provides a computer-implemented method of selecting an ingestible product or combination of ingestible products for a diet, comprising the steps of accessing a purchase list representing the diet's composition of ingestible products over a period of time in terms of product IDs; accessing a nutritional information database to obtain, for each product ID on the purchase list, a nutritional profile representing the amount of each of a plurality of nutrients in the product corresponding to the product ID; combining the purchase list with the nutritional profile for each product ID on the purchase list to obtain a nutritional purchase list, representing the diet's composition of ingestible products over the period of time in terms of the amount of each of the plurality of nutrients; generating a nutrition gap chart representing, for each of the plurality of nutrients, the difference between the nutritional purchase list and a nutritional composition target of the diet for the same period of time; performing a mathematical analysis on data represented by the nutrition gap chart to obtain, for one or more chosen nutrients from among the plurality of nutrients, a required balance profile in dependence upon the size of the difference or relative sizes of the differences; and selecting from an ingestible product database an ingestible product or combination of ingestible products having a nutritional balance profile representing the amount of the one or more chosen nutrients that matches the required balance profile to within a predetermined error margin, and an apparatus for doing the same.

FIGURE 1

EP 2 518 649 A1

## Description

This invention lies in the technical field of food intake analysis, and in particular relates to the nutrient-based selection of ingestible products to satisfy a target nutritional composition of a person or group's food intake.

The cost of healthcare is increasing exponentially; a large part of which is caused by chronic disease management. Until now, we have been largely relying on clinical organisations (e.g. GPs and hospitals) for health management. However, health management is not only about clinical actions, such as diagnosis and treatments. Everyday lifestyle also largely influences people's health. Since clinicians do not necessarily have the time and/or the resources to support preventative activities, new players are expected to play an important role in this prevention market, such as retailers, institutions, or local government.

Although some chronic diseases (i.e. lifestyle diseases) are preventable through lifestyle improvement (e.g. diet, exercise), it is very difficult to achieve lifestyle change. Without any symptoms (e.g. pain, discomfort), people forget about its importance and lose motivation. Therefore it is desirable to have a mechanism to keep motivation up for health management via user engagement.

Current services which are related to prevention of lifestyle diseases can be categorised into the following three areas:

- Exercise & activity
- Food intake
- Weight (& other vital sign) monitoring

Out of those three areas, food intake and the analysis thereof presents a number of technical problems for healthcare organisations or retailers interested in integrating themselves in the health management of their customers. Food intake analysis is conventionally achieved in the following ways:

- Meal plans (recipes): Meal plans require a consumer to follow provided recipes to prepare healthy food. Although this provides much more accurate calorific calculation through an apparently complete knowledge of nutritional intake, it will require the users to change their eating habit completely into specified food types. Therefore users need to buy different ingredients to the usual shopping.
- Food log analysis: Food log analysis consists of the users choosing the food type of the dinner they have eaten for that particular day, and giving a rough quantity of the meal.

Although this enables the dieting system to calculate users' daily intake of calorific value, it requires users to spend some time to record their food diary everyday. This extra effort can discourage users' initiatives, unless they are highly motivated to lose weight or to achieve some other health target linked to food intake.

- Dietician's analysis: Unless we get seriously ill, we tend not to get dieticians' support to analyse our eating habit. This type of analysis is limited in its availability and is likely to still require analysis of food intake via one of the first two methods.

There are several challenges associated with food intake analysis:

One of the challenges associated with improving eating habits is related to the extra effort required by the consumer. Although we can change our lifestyle if we feel the need for change, we tend to go back to the original lifestyle soon after, because the change is too stressful. The mechanisms above require significant user efforts and therefore it is easy for the user to become disengaged. It is easier (less stressful) for consumers to follow new eating habits if it involves less intervention and effort. If it is easier, then the consumer is more likely to carry on, and therefore it is more likely to succeed. Also if there are no immediate benefits for the consumers, they can lose motivation easily.

It is important to note that even for a health management program in which weight-loss is a primary target, it is desirable to ensure that a diet is balanced across a range of nutrients, instead of just looking at reducing calories. Choosing food items for a balanced diet is not a trivial task for people who do not have extensive knowledge of nutrition. Firstly it is necessary for the user to understand their eating habits and identify the nutrients which are lacking. This requires detailed food intake analysis and hence is subject to the problems discussed above. Even after identification of lacking nutrition, it is not just the matter of looking for a product with high value in the lacking nutrition, as if not carefully chosen, this might further impact the food balance. It is necessary to look at the overall balance. Such an analysis is complex and can be intensive in terms of its use of computing resources.

With lack of diet expertise, it is necessary to represent the nutrition information in a way which can be easily understood by the consumer or person responsible for an individual or group's nutritional intake. Currently there is a gap between this easy-to-understand presentation of data and detailed nutrition data. There are initiatives to label products with easy-to-understand nutrition information, such as traffic light labelling. Although these labelling initiatives help consumers become aware of the nutritious content, it is at individual product level and no analysis is being done for the overall food consumption. Due to time limitation, people do not add up all these different pieces of nutritional information, unless they have specific need for it.

There are many ways in which we can obtain advice on dieting. In fact, we can find too much information and we don't know which one to follow. In addition, the advice is usually generic, and not based on collected personal data. Some advice might work for a particular person because of that person's lifestyle, but it might not work for other people. Before making effort to improve lifestyle via health management, it is important for the consumers to be aware of their existing situation to understand the need for change. Therefore, personalisation of advice may be desirable, although collecting personal data and conducting analysis is time-consuming and computationally expensive. Currently, personalised advices tend to be obtained only from dieticians. Therefore unless you get seriously ill and require dietician, we don't get much feedback about the food we eat.

An available mechanism to encourage people to do more physical activities and eat healthy food is to use loyalty cards at retailers to reward their healthy activities. Supermarkets are important places which are related to people's eating habits, since people make decisions about what to eat every time they visit supermarkets, either in-person or online. Compared to clinical organisations, they are also more accessible to people without any symptoms, with much longer opening hours and no requirement to schedule an appointment. Taking advantage of retailers' existing loyalty systems, the accumulated points can be redeemed on goods and/or services in which people are interested (e.g. entertainment) for further encouragement. However, it is desirable to further develop the relationship between consumer health management and retailers.

According to an embodiment of the present invention there is provided a computer-implemented method of selecting an ingestible product or combination of ingestible products for a diet, comprising the steps of accessing a purchase list representing the diet's composition of ingestible products over a period of time in terms of product IDs; accessing a nutritional information database to obtain, for each product ID on the purchase list, a nutritional profile representing the amount of each of a plurality of nutrients in the product corresponding to the product ID; combining the purchase list with the nutritional profile for each product ID on the purchase list to obtain a nutritional purchase list, representing the diet's composition of ingestible products over the period of time in terms of the amount of each of the plurality of nutrients; generating a nutrition gap chart representing, for each of the plurality of nutrients, the difference between the nutritional purchase list and a nutritional composition target of the diet for the same period of time; performing a mathematical analysis on data represented by the nutrition gap chart to obtain, for one or more chosen nutrients from among the plurality of nutrients, a required balance profile in dependence upon the size of the difference or relative sizes of the differences; and selecting from an ingestible product database an ingestible product or combination of ingestible products having a nutritional balance profile representing the amount of the one or more chosen nutrients that matches the required balance profile to within a predetermined error margin.

The computer-implemented method of claim 1 is a computationally efficient technique for selecting ingestible products to achieve or contribute towards achieving a target nutritional composition of a diet. The generation of the nutrition gap chart for use as an analysis tool simplifies the analysis step by focussing on nutrients where differences exist, and by introducing a graphical tool which can be used to analyse the balance between different nutrients.

The combination of the purchase list with the nutritional information enables a nutritional purchase list to be obtained without requiring details of individual items purchased, or selected for future purchase, by the user to be entered into a system manually, and without requiring nutritional details to be input by the user. In making use of the purchase list the computer-implemented method of the present invention thus relies on data which is already available rather than requiring a user to duplicate information. The user may be the person or people whose diet's composition is represented by the purchase list, or may be somebody responsible for the diet of a person or group of people, or somebody responsible for designing the diet of a person or group of people. The user may be a dietician preparing a diet or diet plan for a customer, consumer or patient.

The diet in question may be any collection of ingestible products which can be modified to fulfil a particular nutritional composition target. The diet may be a person or group of people's intake of ingestible products over a particular period of time in the past or in the future, for example, the relevant period of time may be dependent upon whether the purchase list represents past purchases or products selected for future purchases or both. Alternatively a diet may be a dietary plan for a person or group of people, with the purchase list representing ingestible products purchased by or on behalf of that person or group of people in the past or selected for future purchase. The user of the computer-implemented method may be the person whose intake is represented by the diet, or may be an individual preparing a diet for a person or group of people. The computer-implemented method may involve the user interacting at certain points of the method, or providing information used in the method. In a particular implementation, the diet may be the food intake of a family or household on behalf of which ingestible products are purchased together, and optionally linked to a purchasing profile, such as a loyalty card ID. That is to say, the diet may be linked to a profile on a database to which transactions or purchases can be attributed via a profile ID, enabling the purchase list for a diet to be maintained.

The required balance profile is an amount of each of the one or more chosen nutrients and the amounts are selected to remedy the difference or differences represented on the nutrition gap chart. The required balance profile may also be dependent upon other factors such as the timescale over which the nutritional composition target is to be achieved. The nutritional composition target is the goal which the diet should meet. When the diet is a person or group's intake of ingestible products, the nutritional composition target can be considered a nutritional consumption target.

Ingestible products may include food, drink, nutritional supplements, or medication. Representing the diet's composition of ingestible products over the period of time may include listing an amount of each of a plurality of nutrients in the diet. Alternatively, the representation may be in a graphical format, such as a radar chart, with an axis or spoke for each nutrient. The difference between the nutritional purchase list and the nutritional composition target may be represented in the same way.

The predetermined error margin may be as small as zero if a precise match between balance profiles is required. A balance profile may be simply a list of component nutrient amounts, or may be a property of a graphical representation of amounts of nutrients. The predetermined error margin may vary depending on the particular implementation.

Optionally, the number of chosen nutrients is two or more. Advantageously, the computer-implemented method of the present invention is able to handle scenarios where several nutrients need to be considered in order to meet a nutritional composition target in an analytically simple and computationally efficient manner. The generation of the nutrition gap chart and the use of the required balance profile means that the number of nutrients on whose differences the required balance profile is based can be increased without a significant increase in the computational resources required to select an ingestible product or combination of products.

Embodiments of the present invention may further comprise displaying the nutrition gap chart as a radar chart. The nutrition gap chart may be displayed to the user of the computer-implemented method. A radar chart is a convenient presentation mode for the nutrition gap chart because it allows a user to assess to view the differences that exist between their nutritional composition target and their nutritional purchase list for a number of nutrients at once. Furthermore, properties of the radar chart can be used in the mathematical analysis step to ensure that the mathematical analysis is computationally efficient.

The purchase list may be averaged over the period of time during which the purchases were made or are being made for in order to give a more representative view of a diet's nutritional composition. In terms of being easy to understand for a user, in embodiments of the present invention the nutritional purchase list and the nutritional composition target are scaled with respect to the period of time for which the diet's composition of ingestible products is represented by the purchase list so that the nutrition gap chart represents an arbitrary time period. The arbitrary time period could be, for example, one day or one week. It is common in the technical field of nutritional data analysis to analyse nutritional composition of a diet with respect to an amount of a nutrient recommended for daily consumption by a regulatory authority. In the UK, for example, the 'Recommended Daily Amount' of a nutrient is set by the Food Standards Agency. In the weekly example the daily amount is simply multiplied by 7. Nutritional information is often provided as a proportion of the recommended daily amount, and the nutritional profile in embodiments of the present invention may be provided in such a format. Displaying the nutrition gap chart with the axes graded and normalised with respect to the recommended daily amount is convenient and informative for a user. Other criteria other than Recommended Daily allowances could be used by the user in order to set his/her own criteria for nutritional goals for the diet.

Recommended daily allowances (or recommend daily amounts) and other such advisory information provides a useful default nutritional composition target. However, nutritional requirements and health considerations vary from diet to diet. Embodiments of the present invention may further comprise setting the nutritional composition target of the diet based on diet-specific information. Embodiments of the present invention are not restricted to prescribed nutritional composition targets, and some level of personalisation or individualisation may be possible depending on the embodiment. For example, whether the diet is for a male or female (or the ratio of males to females in a group) may determine a target calorific intake per day, the number of people whose food intake is represented by the diet, for example in the case of a family or household, may change the nutritional composition targets. Furthermore, health information may be taken into account, so that conditions which require increased or reduced consumption of particular nutrients are taken into account. Alternatively, a user may be able to configure the nutritional composition target directly. The diet-specific information may be stored in a customer data storage unit, or may be input by a user for the purpose of setting or modifying a nutritional composition target. Whilst meeting a nutritional composition target may be important for a diet, there are some considerations, such as a desire for a certain item over another, which are unpredictable and cannot be taken into account in the selection method without user input. To cater for such scenarios, two or more ingestible products or combinations of ingestible products are selected from the ingestible product database, each selected product or combination of products having a nutritional balance profile representing the amount of the one or more chosen nutrients that matches the required balance profile to within a predetermined error margin. Selecting two or more different ingestible products or combinations of ingestible products means that the selected products can be displayed to the user in a way which allows the user to choose a particular one of the two or more ingestible products or combinations of ingestible products to purchase and thus be introduced to the diet in future.

Optionally, the computer-implemented method is performed in response to a request from a user of a web-tool or other program.

The method embodying the present invention can be used to select products even in the absence of any data about past purchases. Optionally, the nutrition gap chart is generated in response to a request from a user, the purchase list includes a list of future purchases, and the method further comprises responding to the request by displaying a graphical representation of the future nutrition gap chart. Future purchases are ingestible products that the user has indicated may be purchased, for example by selection for and placement in a physical or virtual shopping basket/trolley, or based on a projection of previous purchase lists. The future nutritional gap chart enables the user to see the effect that the ingestible products in the purchase list will have on the nutritional composition of the diet. Furthermore, upon execution of the method, a user will be able to explore the effect on the nutritional composition of the diet with respect to a nutritional composition target of adding, subtracting, or substituting certain ingestible products from the future purchase list.

Alternatively, the nutrition gap chart is generated in response to a request from the user, the purchase list includes a list of past purchases, and the method further comprises responding to the request by displaying a graphical representation of the past nutrition gap chart. Past purchases are those ingestible products that have been purchased as part of a diet. The use of a profile ID as discussed above may be an appropriate way of attributing purchases or transactions to a particular diet (i.e. to the diet of a particular group/individual/time period). Selecting ingestible products based on analysis of past purchases means that an indication of those products can be displayed to the user as a way of remedying the differences between the diet's past nutritional composition and target nutritional composition.

As a further alternative, the nutrition gap chart is generated in response to a request from the user, and the purchase list includes both past and future purchases. For example, a time period of a month may be used as a time period to gather purchase list data for a particular diet, and that time period may include a forthcoming transaction or set of purchases.

A request by a user may be in the form of an http request or request made via a thin client to a server. The server may respond with a representation of the nutrition gap chart and an indication of the selected ingestible product or ingestible products. The user could send such a request at any time in order to ascertain how the diet's nutritional composition (based on the nutritional purchase list) compares with the target nutritional composition for that diet, and optionally to receive an indication of selected ingestible products appropriate to remedy differences represented on the nutritional gap chart. Optionally, the response to the user's request further comprises information identifying the one or more selected ingestible product or combination of ingestible products.

In responding to a request from a user, particularly when two or more ingestible products or combinations of ingestible products are selected, the method may include grading each of the one or more ingestible products or combinations of ingestible products according to an algorithm. The details of the algorithm will depend on the particular requirements of the individual implementation of the invention. However, the algorithm is at least based on based on the amount of the one or more chosen nutrients present in the selected ingestible product or combination of ingestible products relative to the sizes of the differences for the chosen nutrients. For example, once the required balance profile is established, an ingestible product or combination of ingestible products that closely matches the required balance profile may be graded above one which only loosely matches the required balance profile. Additional factors such as the balance of other nutrients in the selected ingestible product or combination of ingestible products may be taken into account. Diet-specific information may be used by the algorithm, for example, if the diet's purchase history indicates a preference for a particular type of ingestible product over another (for example fresh food over dietary supplements) then that may be reflected in the grading.

The algorithm may be used as a discriminator so that only, for example, the top three ingestible products or combination of ingestible products are displayed. Alternatively, the algorithm may incorporate a scoring metric, and a threshold score is used as a discriminator so that only ingestible products or combinations of ingestible products exceeding the threshold are displayed to the user. Displaying information to the user involves the transfer of data, for example between a server and client, therefore grading enables efficient data exchange by only displaying details of ingestible products or combinations of ingestible products that are likely to appeal to the user (since the user is likely to request more selections until a suitable selection is displayed).

The required balance profile is not necessarily equal to the sizes of the differences. Optionally, the required balance profile is also dependent upon a time moderation factor representing a length of time by the end of which the diet's nutritional composition target is to be met. It may be advantageous to gradually increase the amount of a nutrient in a diet's nutritional composition rather than simply selecting an ingestible product having the right amount of each of the chosen nutrients to remedy the difference or differences.

Basing the nutritional composition analysis of a diet on a purchase list reduces the burden on the user in terms of keeping a record of nutritional consumption. This ensures that the data is kept up to date. Optionally, the purchase list for the diet is updated by the user or by a retailer linking a transaction identifying one or more product IDs to the diet. The burden on the user and therefore the system as a whole is reduced. This ensures that the method is efficient in terms of the amount of data that is transferred from one step of the method to another.

The mathematical analysis process and ingestible food product selection process may vary depending on the particular embodiment. Optionally, the computer-implemented method embodying the present invention includes the following steps: generating a radar chart as the nutrition gap chart; for each difference represented on the radar chart, generating an x-y coordinate corresponding to its position on the radar chart; conducting a graphical analysis of the x-y coordinates to obtain a required radar balance profile as the required balance profile; and selecting an ingestible product or combination of ingestible products by, for each of a number of potential ingestible products or combinations of ingestible products, generating a series of x-y coordinates corresponding to the position on a radar chart of the amount of the one or more chosen nutrients, conducting a graphical analysis of the series of x-y coordinates to obtain a potential product radar balance profile, comparing the required radar balance profile with the potential product radar balance profile, and assessing how closely they match. The graphical analysis technique will depend on the particular embodiment, but may be, for example, calculating the gradient between x-y coordinates for neighbouring nutrients on the radar chart, or calculating the area bounded by a triangle joining neighbouring points on the radar chart with the origin. The 'radar balance profile', which term encompasses both the required radar balance profile and the potential product radar balance profile, is introduced as a new parameter.

The radar balance profile parameter facilitates efficient comparison of the nutritional balance of the potential products with the required nutritional balance. Radar balance profile characterises the balance of nutrition by analysing a nutritional radar chart graphically. This enables efficient comparison of nutritional balance, particularly in embodiments in which the nutritional information database also stores nutritional profile information with radar balance profile data.

Conventionally the product recommendation for a nutrient lacking from a diet's nutritional composition is achieved by listing up products which have high values in one particular nutrition group. With this method, however, it is difficult to analyse the overall balance of the nutrition of the products, since it is necessary to analyse their relative values in addition to the absolute nutritional values, which increase the complexity and therefore required computational time and power.

The balance profile of an ingestible product is important because, for example, it is not advisable to recommend to somebody, who has insufficient calcium intake, something which is high in calcium and also high in energy, if that person is trying to lose weight.

A benefit of introducing radar balance profile is that it can focus on the nutritional balance, rather than absolute values of nutrition. By converting nutritional values expressed in a radar chart into x-y coordinates, this method reduces the calculation complexity and therefore reduces the calculation power and time required.

The present invention may also be embodied as an apparatus for selecting an ingestible product or combination of ingestible products for a diet, comprising: a purchase list access unit configured to access a purchase list representing the diet's composition of ingestible products over a period of time in terms of product IDs; a nutritional information database access unit configured to access a nutritional information database to obtain, for each product ID on the purchase list, a nutritional profile representing the amount of each of a plurality of nutrients in the product corresponding to the product ID; a combination unit configured to combine the purchase list with the nutritional profile for each product ID on the purchase list to obtain a nutritional purchase list, representing the diet's composition of ingestible products over the period of time in terms of the amount of each of the plurality of nutrients; a nutrition gap chart generating unit configured to generate a nutrition gap chart representing, for each of the plurality of nutrients, the difference between the nutritional purchase list and a nutritional composition target of the diet for the same period of time; an analysis unit configured to perform a mathematical analysis on data represented by the nutrition gap chart to obtain, for one or more chosen nutrients from among the plurality of nutrients, a required balance profile in dependence upon the size of the difference or relative sizes of the differences; an ingestible product selection unit configured to select from an ingestible product database an ingestible product or combination of ingestible products having a nutritional balance profile representing the amount of the one or more chosen nutrients that matches the required balance profile to within a predetermined error margin.

A detailed description of some embodiments of the present invention will now be provided, purely by way of example, with reference to the attached drawings in which:

Figure 1 is a block diagram of an apparatus embodying the present invention;
Figure 2 illustrates a sample hardware configuration of an apparatus embodying the present invention;
Figure 3 illustrates an example of a radar chart comparing a diet's average nutritional composition to a target nutritional composition in terms of percentage of RDA consumed per day;
Figure 4 illustrates how a nutrition gap value chart can be generated from the differences between current composition amount (based on past nutritional purchase list) and a target composition amount;
Figure 5A illustrates a radar chart showing gap values for a number of nutrients, and Figure 5B shows the corresponding components of the required balance profile on a radar chart with the same scale and axes for comparison;
Figure 6 illustrates the superposition of an x-y coordinates system over a radar chart to form the basis of the x-y coordinates used in producing the radar balance profiles;
Figure 7 illustrates two ways in which the nutrition balance can be derived from nutrition radar charts to obtain a radar balance profile;
Figure 8 is a chart illustrating calculations for use in calculating gradient information when obtaining a radar balance profile;
Figure 9 illustrates the integral calculus involved in an alternative technique for obtaining a radar balance profile;
Figure 10 illustrates some alternative cases in which the integral calculus varies from that shown in Figure 9;
Figure 11 illustrates an example of a radar chart representing a required balance profile for nutrients which are currently lacking from the nutritional purchase list, and some potential products selected to remedy the lack of nutrients;
Figure 12 illustrates an example of a radar chart showing nutrients that are present in excess in the nutritional purchase list, and a product from the purchase list that has been identified for replacement by a selected product to remedy the excess;
Figure 13 shows a block diagram of an apparatus embodying the present invention;
Figure 14 is a flowchart showing the process performed by the apparatus of Figure 13; and Figure 15 shows, in a radar chart format, the incremental steps by which the differences in the nutrition gap chart can be gradually reduced.

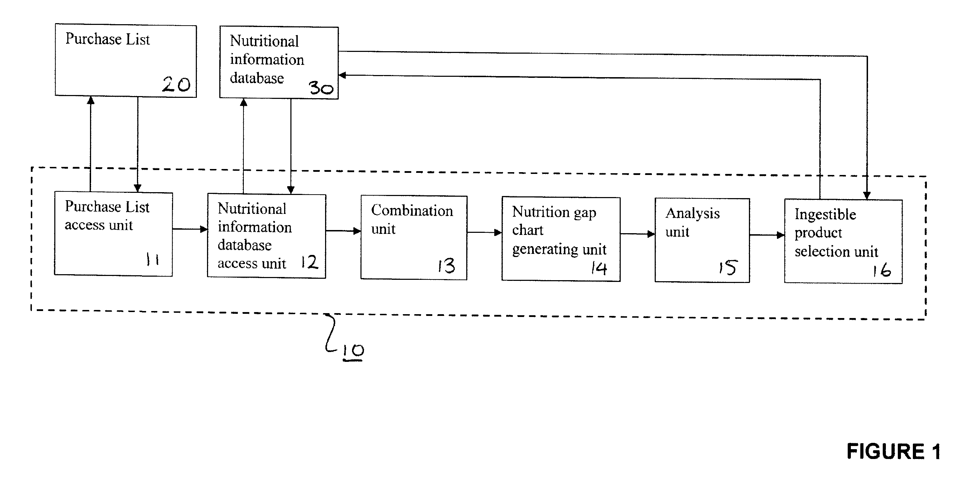

Figure 1 is a block diagram of an apparatus 10 embodying the present invention. In a particular implementation, the embodiment is merely those components falling within the dashed line. However, embodiments of the present invention may include the purchase list 20 and nutritional information database 30 as well.

The apparatus 10 includes a purchase list access unit 11, a nutritional information database access unit 12, a combination unit 13, a nutrition gap chart generating unit 14, an analysis unit 15, and an ingestible product selection unit 16. The apparatus also represents the configuration of an apparatus suitable for performing the computer-implemented method embodying the present invention. In such embodiments, the apparatus 10 is a computer, and each unit within the computer is a functional unit comprising executable code for execution by a processor, and possibly also having the functionality to employ other components of the computer, such as a network interface card, memory and storage. Furthermore, it is of course possible that the method, or even each step, could be carried out by a plurality of computers.

The purchase list access unit 11 has data connections to the purchase list 20 and the nutritional information database access unit 12. The purchase list access unit 11 is operable to send a request to the purchase list in order to obtain a list representing a diet's composition over a period of time in terms of product IDs. The purchase list access unit 11 is then operable to transfer the list so obtained to the nutritional information database access unit 12.

The nutritional information database access unit 12 is operable to send a request to the nutritional information database 30. The request may contain the list received from the purchase list access unit 11, or the nutritional information database access unit 12 may be operable to modify the list to extract certain required data in order to compile a request which at least contains information which the nutritional information database 30 can use to identify the products for which a nutritional profile is required.

The nutritional information database 30 is operable to respond to a request from the nutritional information database access unit 12 with a nutritional profile for each product identified in the request. Products may be identified, for example, by a product ID system in which each product available at a retailer has a unique ID.

The combination unit 13 is illustrated as a functionally separate unit. It should be understood that the function of the combination unit 13 could be performed by the nutritional information database access unit 12 in receiving the nutritional profiles from the nutritional information database 30. The combination unit 13 is operable to receive the purchase list expressed in terms of product IDs, and the nutritional profile for each product ID on the purchase list, and to combine them to obtain a nutritional purchase list, representing the diet's composition of ingestible products over the period of time in terms of the amount of each of a plurality of nutrients. The combination unit 13 may be operable to incorporate a wastage factor into the combination, so that a percentage of purchase food that is typically wasted rather than consumed is taken into account.

The nutrition gap chart generating unit 14 may store a nutritional composition target for a diet over a particular period of time. Alternatively, the nutrition gap chart generating unit 14 may be operable to access a remote customer data storage unit in order to access a nutritional composition target for the diet. The nutrition gap chart generating unit 14 is also operable to receive the nutritional purchase list from the combination unit 13. The nutrition gap chart generating unit 14 may be operable to moderate the nutritional purchase list and/or the nutritional composition target so that they represent the same period of time. The nutrition gap chart generating unit 14 is then operable to generate a nutrition gap chart representing the difference between the nutritional purchase list and a nutritional composition target over the relevant period of time. The differences may be positive or negative values. The chart may be, for example, a bar chart, a pie chart, or a radar chart. Once the nutrition gap chart is generated it is ready to be transferred to another apparatus or component in response to a request. The destination of the nutrition gap chart is implementation-specific, but it may be transferred to a client-side device and displayed on a display unit, or reproduced in printed form.

The analysis unit 15 is operable to receive from the nutrition gap chart generating unit 14 either the nutrition gap chart or the data represented by the nutrition gap chart, depending on the particular implementation. The analysis unit 15 and ingestible product selection unit 16 may operate on a nutrient-by-nutrient basis to select ingestible items which will result in an aggregate nutritional balance that is closer to the nutritional composition target than the nutritional purchase list. Alternatively, the analysis unit 15 and ingestible product selection unit may operate on groups of nutrients or the entire range of nutrients for which information is available. Based on the size of the difference or the relative sizes between the differences for each of the chosen nutrients, the analysis unit is operable to obtain a required balance profile. Optionally, the analysis unit 15 may be operable to take into account the period of time over which the diet's nutritional composition target is to be met, so that the required balance profile represents a balance between nutrients which a selected ingestible product should contain, as an addition to or as a substitution into the diet's composition of ingestible products, in order to reduce the size of the difference or sizes of the differences.

The analysis unit 15 and ingestible product selection unit 16 may be operable to feedback to the combination unit 13 so that certain ingestible products can be removed from the nutritional purchase list and replaced by a product selected by the ingestible product selection unit 16 for the nutrition gap chart generation and subsequent analysis.

The ingestible product selection unit 16 is operable to receive the required balance profile from the analysis unit 15. In the example of Figure 1 the ingestible product selection unit 16 is shown with a data link to the nutritional information database. In such an arrangement, the ingestible product selection unit 16 is operable to submit a request to the nutritional information database detailing the required balance profile, in response to which the nutritional information database will respond with data identifying an ingestible product or combination of products that match the required balance profile, or match it to within a predefined acceptable error margin. Alternatively, the ingestible product selection unit may have access to a distinct database. The ingestible product selection unit may be able to access the purchase list and/or an external customer data storage unit in order to access data for assessing the suitability of selections.

Once the ingestible product selection unit 16 has selected one or more ingestible products or combinations of ingestible products, data identifying those ingestible products can be transferred to another device or component. The destination of such data will depend on the particular implementation, but could be, for example, a client device, a retailer's own system so that the data can be used on a checkout receipt, or the combination unit 13 so that the effect of the hypothetical inclusion of the selected ingestible product or combination of ingestible products on the nutrition gap chart can be considered.

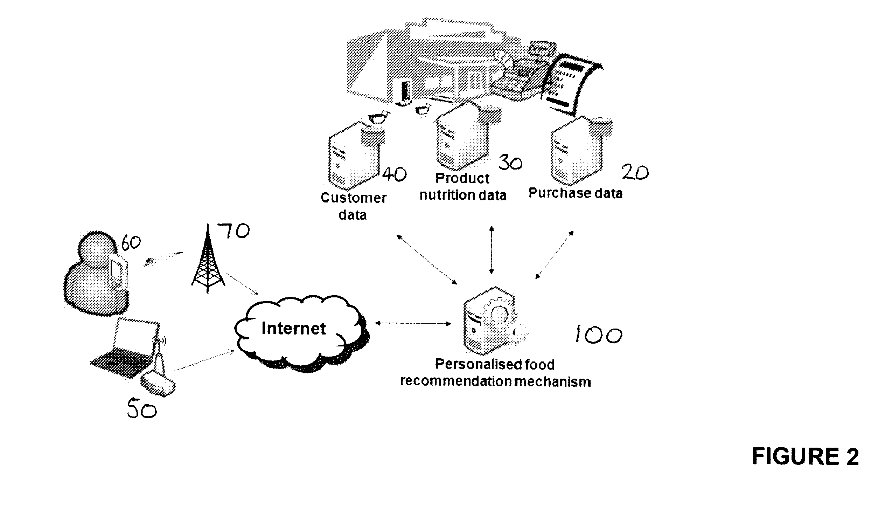

Figure 2 illustrates a sample hardware configuration of an apparatus embodying the present invention, or a computer system on which a computer-implemented method embodying the present invention could be performed.

In Figure 2 the purchase data storage unit 20 and product nutrition data storage unit 30 are same as the purchase list 20 and nutritional information database 30 in Figure 1 respectively. The customer data storage unit 40 is also provided as an external storage unit. The personalised food recommendation mechanism 100 is a server-style computer which has equivalent functionality to the apparatus 10 in Figure 1. The personalised food recommendation mechanism is operable to exchange data with the purchase data storage unit 20, the product nutrition data storage unit 30, and the customer data storage unit 40.

The personalised food recommendation mechanism 100 is accessible to a user as a web tool, for example as a GUI, by using a personal computer 50 over the internet, or by using a mobile telecommunications terminal 60 over a radio network 70.

An embodiment of the present invention will now be described. For this embodiment, three types of data are required, which are:

History of customer purchase data:

- Purchase items with product ID
- Quantity of purchased items (e.g. 130g)
- Purchase date

Product nutrition data:

- Quantity of the product per item (e.g. 500g per pack)
- Nutrition information per 100 grams

Customer profile data (diet):

- Number of family members/people linked to loyalty card profile
- User/diet profile ID (patient number/loyalty card number etc)

From the customer profile data (stored in a customer data storage unit) of the scheme, which may be a retailer's loyalty card scheme, a retailer-independent third party scheme for recommending and selecting retailer's products for a diet, or a healthcare scheme run by a healthcare provider such as the NHS (which may also fit the previous category of retailer-independent third party schemes), the number of people for whom purchases are linked to the relevant profile ID may be identified. In some schemes, this may necessarily be a single person. Alternatively, information regarding the number of people linked to a profile ID may be unavailable and it may simply be assumed that the profile ID is linked to, for example, a single person. Alternatively, for example in the loyalty card scheme, the profile ID may be linked to a family, hence associated data regarding the size of family linked to that loyalty card may be available. As food shopping is usually done for a family, the purchase data (purchase list) includes food which is consumed by the family, rather than one individual, unless a person is living alone. Therefore, in such an implementation, it is necessary to divide the portion by the number of family members. The family unit is appropriate for consideration, as a family may be mutually responsible for each other's health. If a person is living alone, then the purchase data can be used as it is for diet composition analysis for that person. In this embodiment, the 'diet' is the diet of whoever is linked to the profile ID. Controller is used to refer to the retailer, third party, healthcare provider, or any other party responsible for administering the overall scheme.

In implementations in which the profile ID can represent the nutritional composition of the diets of more than one person, the user may be able to, for example via a GUI or web tool, personalise the nutritional composition data and nutritional composition targets for a particular diet in a way other than simply dividing the total amount purchased on a per head basis. It may be that a particular person's diet has a personal nutritional composition target and, for example, their nutritional composition can be input by a tool allowing the user to manipulate the purchase list by allocating purchases (or portions of purchases) on the purchase list to that person.

Any other additional personal data can be provided by customers through a web tool (for example, a GUI embodying the present invention) in order to increase the quality of service and for further personalisation.

Before the embodiment can function, it is necessary to build a nutritional information database with a nutritional profile for each product ID. For pre-packaged products with nutrition labels, nutrition information is put into the database with product ID. The availability of the nutrition information depends on the information provided by the suppliers. Therefore the availability of nutritional information may depend on how much was made available by the suppliers. For non pre-packaged products, standard nutrition information for that particular product (e.g. an apple) is used. Once this nutritional information database is completed, the nutritional profile, which may take the form of a chart, or merely a series of values of amounts of particular nutrients, for each product ID is created. Therefore each product ID has nutrition information in the nutritional information database at least in the form of numerical values, and possibly also as a characterisation value, or characterisation values, for the nutrition balance, which will be explained below. The nutrition values are based on the quantity which that particular product package provides, rather than per 100g.

As an initial step, from the diet purchase data, ingestible products are separated from non-ingestible items by a food separator, which is exemplary of a purchase list access unit 11.

The total amount of nutrition (nutritional purchase list) is calculated at the combination unit 13 from food items of the purchase history representing the diet's composition, and using the nutritional profile per product ID from the nutritional information database. In order to take into account consumption of long-lasting products, this total composition is then divided by the number of weeks which the purchase history covers. Therefore average nutrition composition of a diet is given by:

$$[\text{Average Nutritional Composition}] = [\text{Total Composition}] / [\text{weeks of purchase history}].$$

Not all the purchased food will be consumed as part of the particular diet in question. Come of the purchased items may be shared with visitors, or some foods might be thrown away. Therefore, in this embodiment, the statistical data on food wastage is applied. In the UK, for example, households tend to throw away 18% of all food purchased and for a family with children, they tend to throw away 27% of all food purchased. Of those thrown away, bakery good makes up 19% of all avoidable food waste and vegetables contributes to 18%. These values may be configurable by the user or by the controller.

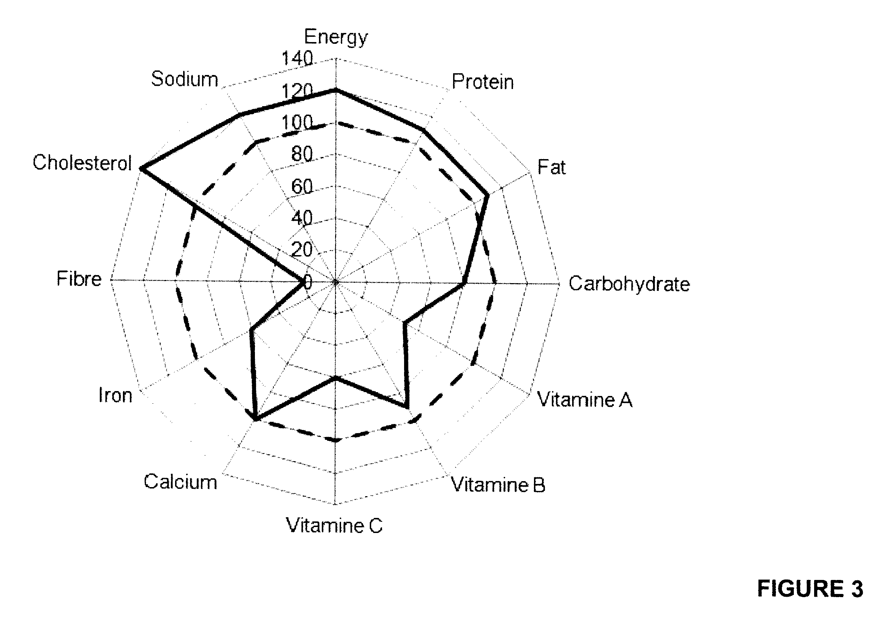

Figure 3 illustrates an example of a radar chart comparing a diet's average nutritional composition (solid line) to a target nutritional composition (dotted line) in terms of percentage of RDA per day. In the example, the target nutrition composition is 100% of RDA per day for each nutrient.

The gap values (differences) are derived from the difference between the target nutritional composition which the diet will ideally contain, and the current composition (i.e. average nutritional composition) by the nutrition gap chart generating unit, which in this example includes a gap value calculator. The target nutritional composition of the diet can be linked to a health goal, for example, losing weight, reducing cholesterol, balanced diet etc, which is then translated into a quantitative goal, which is the target nutritional composition. The target nutritional composition may be expressed as a target amount over a particular time period on a per-nutrient basis. Without users specifying specific goals for a diet, the default goal may be set to be recommended nutrition values, such as Recommended Daily Allowance (RDA). Even if the diet has a goal related to a specific nutrient, embodiments of the present invention conduct analysis in such a way that both the goal of that specific nutrient and also a balanced diet can be achieved, unless a diet has a specific requirement to avoid achieving RDA values for certain nutrients (e.g. for people with diabetes).

The Gap Values are given by:

$$[\text{Gap Values}] = [\text{Goal}] - [\text{Average Nutritional Composition}].$$

Therefore Gap Values are expressed in positive values for lacking nutrients, and Gap Values are expressed in negative values for nutrients consumed in excess.

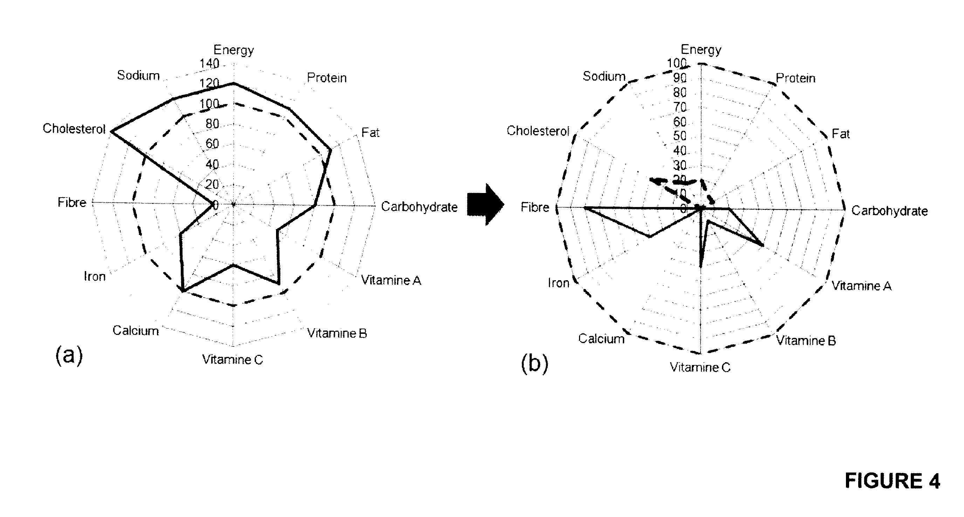

These values are then presented in the nutrition chart, as shown in Figure 4. Figure 4a is the same as Figure 3. Figure 4b shows the nutrition gap chart based on the same nutritional composition data and target nutritional composition data as Figure 3. The lightly dashed line around the outside represents the target nutritional composition, the solid line indicates the insufficient nutrition levels and the thicker dashed line indicates excessive nutrition levels.

Figure 4 illustrates how a nutrition gap value chart can be generated from the differences between current composition (based on past nutritional purchase list) and a target composition amount.

In order to promote sustainable change management by reducing the stress level incurred by the diet when implementing the dietary change caused by purchasing the ingestible products selected by the computer-implemented method embodying the present invention, the gap value is further broken down to smaller suitable incremental steps. The gap value is broken down at the analysis unit 15 in calculating the required balance profile.

The component of the required balance profile for a nutrient is given by:

$$[\text{Component of Required Balance Profile}] = ([\text{Gap Value}] / [\text{Coefficient}]) \times [\text{Engagement Level}]$$

where coefficient is a number which is related to the time period in which the diet's composition should achieve the goal. If the desired time period is not given by the user, then it may be a set value, or may be derived based on knowledge of the person whose diet is being modified.

In this embodiment, the size of incremental step is also related to the level of engagement. If the user frequently purchases products selected by an embodiment of the present invention as part of the diet in question, then the engagement level is high and therefore the incremental steps can be larger. On the other hand, if the diet is rarely modified on the basis of such selections, the engagement level is low and the incremental step remains small. Information relating to engagement level can be stored in the customer data storage unit.

Optionally, for analysis purposes or for displaying to a user, the required balance profile for the chosen nutrients can be displayed on a chart, for example, a radar chart.

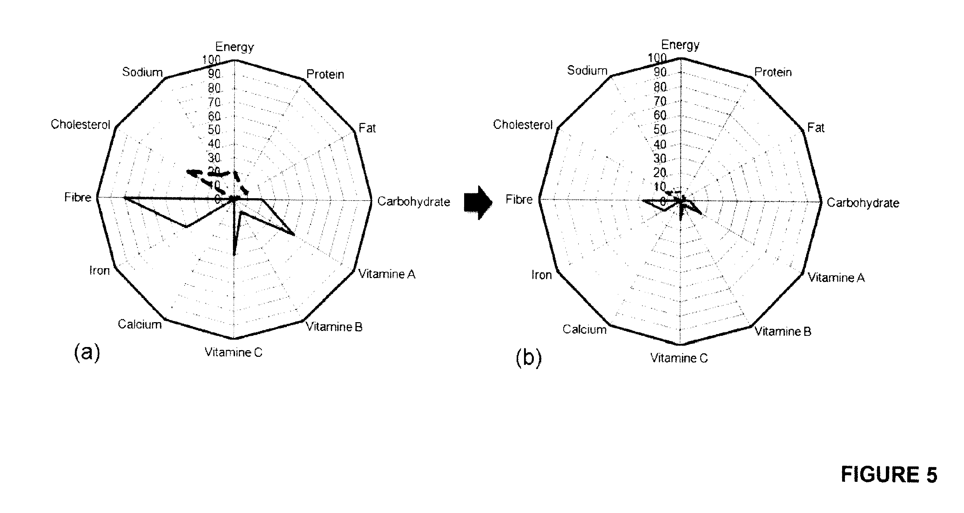

Figure 5A illustrates a radar chart showing gap values for a number of nutrients, and Figure 5B shows the corresponding components of the required balance profile on a radar chart with the same scale and axes for comparison. The lightly dashed line around the outside represents the target nutritional composition, the solid line indicates the insufficient nutrition levels and the thicker dashed line indicates excessive nutrition levels. The shapes defined by the lines is the same in either case, only the size of the shapes has changed, to reflect the calculations performed by the analysis unit 15 in breaking the overall target nutritional composition into smaller steps.

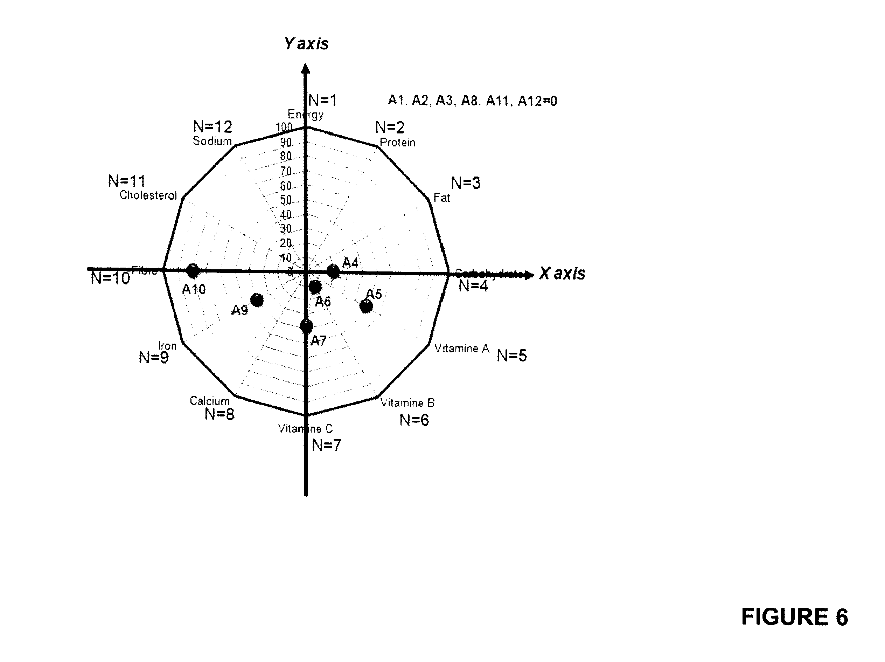

The present embodiment adopts the parameter 'radar balance profiles' for the required balance profile and the potential product balance profiles. Figure 6 illustrates the superposition of an x-y coordinates system over a radar chart to form the basis of the x-y coordinates used in producing the radar balance profiles.

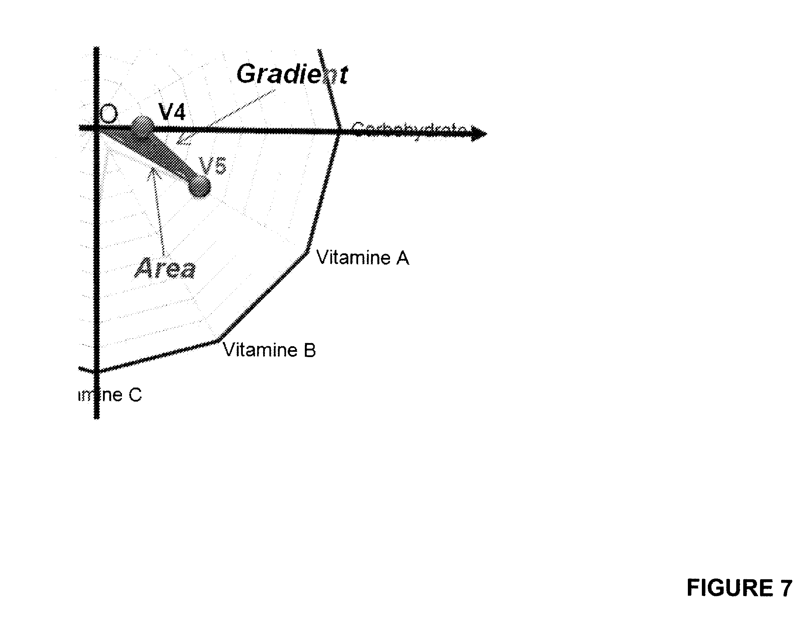

There are two ways in which the nutrition balance can be derived from nutrition radar charts to obtain a radar balance profile, as shown in Figure 7:

a) Gradient of the line between adjacent nutrition values
b) Enclosed area.

Figure 7 illustrates the concepts of (a) Gradient of the line between adjacent nutrition values, and (b) area enclosed by a triangle joining adjacent nutrition values and the origin.

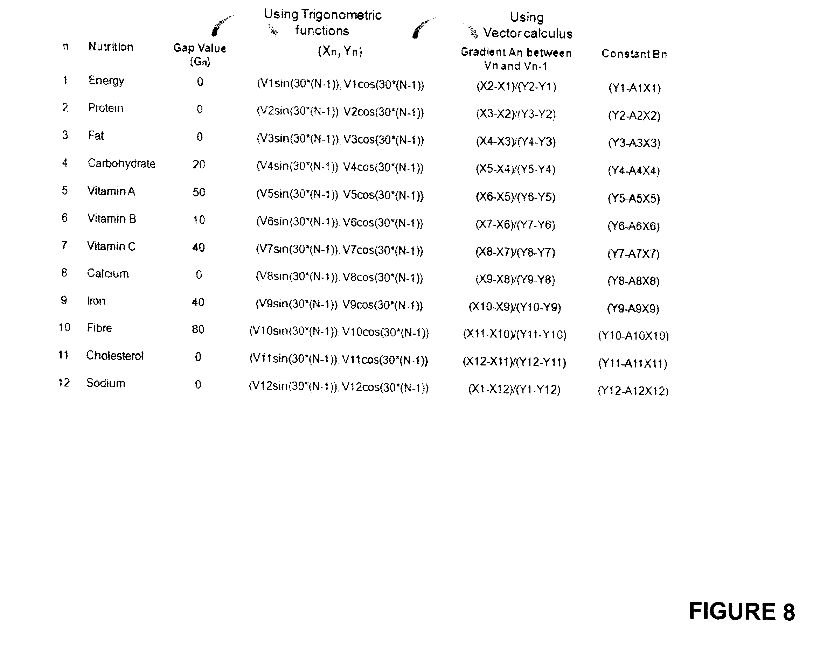

Using Trigonometric functions, the gap values on the radar chart can be converted into points in x-y coordinates, expressed by $(X_n, Y_n)$. The conversion and calculations are shown in Figure 8. This conversion is applied to all nutrition values. The gradient An of the line between the adjacent values are then calculated by:

$$A_n = (X_n - X_{n-1})/(Y_n - Y_{n-1}).$$

An equation for a linear function can be expressed by:

$$y = ax + b$$

where a is a gradient and b is a constant. On the basis of a coordinate on the line and the gradient, the value b can be calculated. Therefore the value b can be calculated for all lines between adjacent nutrition values (amount of a particular nutrient).

The gradient An characterises the nutritional balance of a particular product for at least two adjacent nutrients. For more than two nutrients, more gradients An can be considered. Therefore the recommended products can be found by comparing the gradient(s) of the required radar balance profile with the corresponding gradient(s) of the potential products. Since the gradients might not match exactly, a predetermined error may be incorporated, which may be zero at one extreme.

The nutritional balance can also be characterised by the enclosed area. This is achieved by firstly integrating the linear function between the nutrition values:

$$\int f(x)\ dx$$

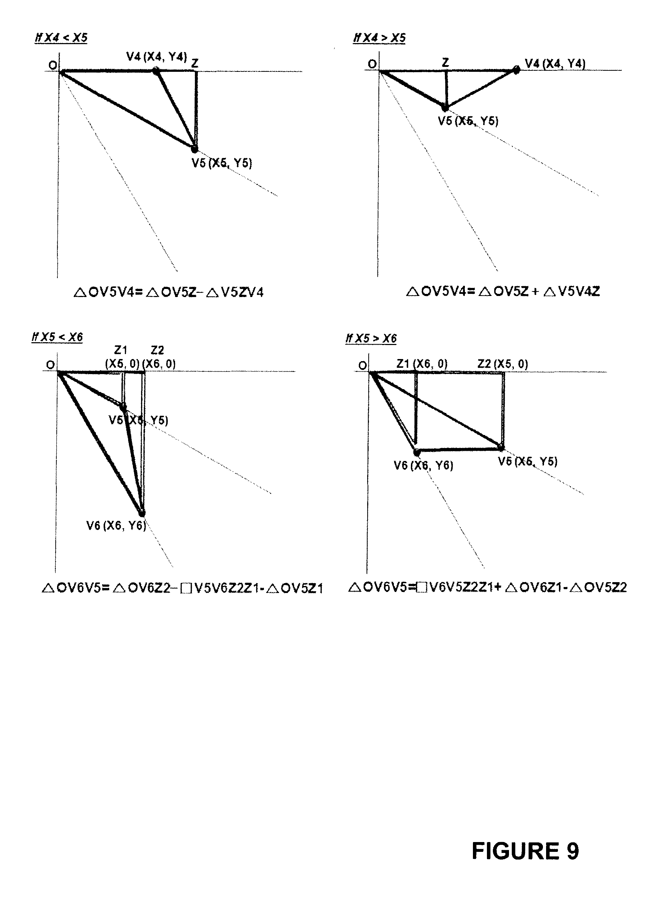

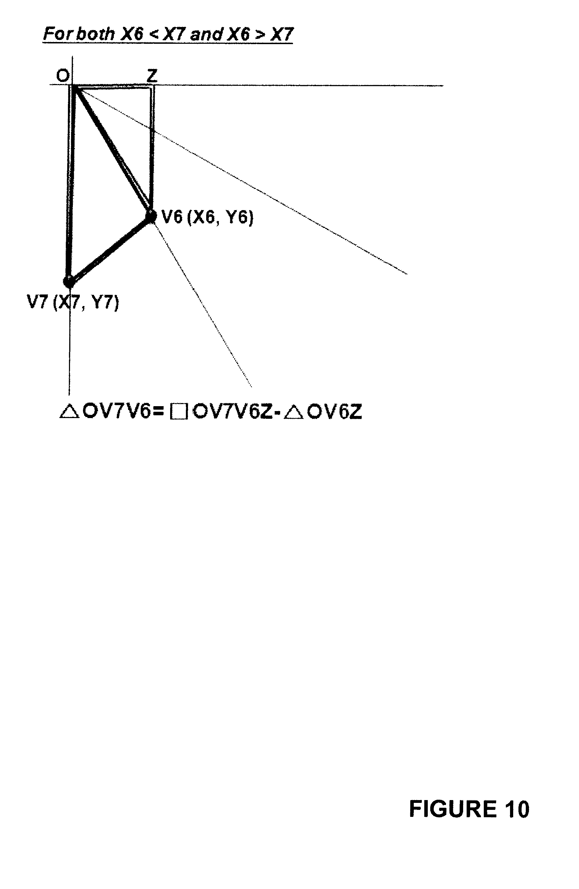

In addition to the integration of the linear function between the nutrition values, there are other integrations needed, as shown in Figures 9 and 10. Figures 9 and 10 illustrate the areas which must be calculated in order to find the area enclosed by the triangle joining adjacent points on the radar chart and the origin. Of course, the method is not restricted to adjacent nutrients, and any pair of nutrients can be compared in this way.

The enclosed area characterises the nutritional balance between two nutrients. Therefore it is possible to compare between the enclosed areas for the required balance profile and those of the potential products in order to identify recommended products.

At the ingestible product selection unit 16, the process of selecting ingestible products to be recommend to be included in the diet. Together with the overall balance of the nutrition, the nutrient most lacking in terms of the difference between the nutritional purchase list and the target nutritional composition is identified and this most lacking nutrient is made a priority nutrient. Alternatively, the priority nutrient may be the nutrient which is being consumed to the greatest excess. Which of the former or latter is made a priority may be diet-configurable, or may be target-dependent. For example, if weight loss is a goal, then reducing the consumption of a nutrient being consumed to the greatest excess may be the priority. If a more balanced diet is the goal, then the priority may be increasing consumption of nutrients most lacking from the nutritional purchase list at present. Therefore, in this embodiment there are two parameters for identifying recommended products: (a) radar balance profile values, and (b) priority nutrient.

Firstly the radar balance profile values of the required balance profile and the potential products are compared. Consequently the candidate products are then graded according to the closeness of their potential product radar balance profile compared to the required radar balance profile, and also by the amount of the priority nutrient (with the goal of achieving target nutritional composition for the priority nutrient above the others). An incentive, such as a larger amount of loyalty points may be offered for those products which are closer to a radar balance profile match and have a larger amount of the most lacking nutrient.

The required radar balance profile can be achieved by either replacing existing ingestible products with new ingestible products, or simply adding new ingestible products, to supply lacking nutrients, or by removing or replacing ingestible products to reduce the amounts of a nutrient showing an excess on the nutrition gap chart.

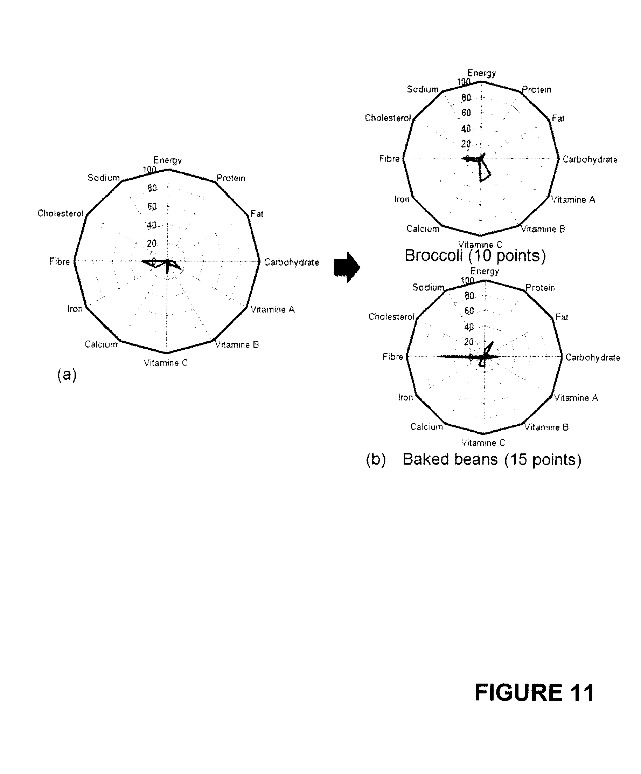

Figure 11 illustrates an example of a radar chart representing a required balance profile for nutrients which are currently lacking from the nutritional purchase list, and some potential products selected to remedy the lack of nutrients.

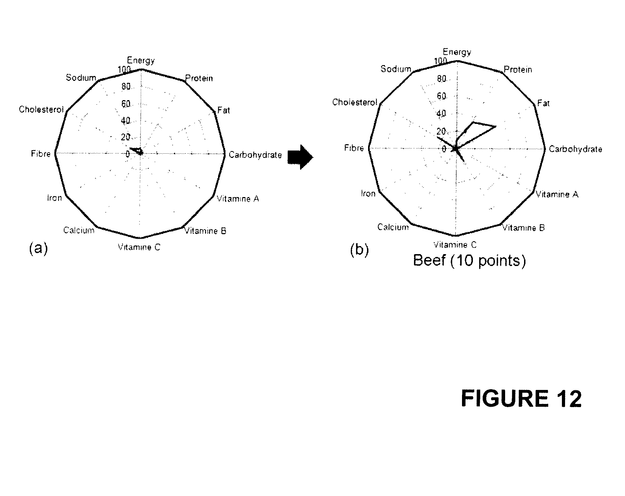

Figure 12 illustrates an example of a radar chart showing nutrients that are present in excess in the nutritional purchase list, and a product from the purchase list that has been identified for removal, or for replacement by a selected product to remedy the excess.

The candidate products that are selected are stored, for example in a customer data storage unit, and recommended to the user, potentially with an offer of an incentive such as money, a discount voucher, or points towards a scheme such as extra loyalty points. Therefore if the user purchases those recommended products to modify the diet towards the goal, not only are they taking a step towards more balanced diet, but they also receive a reward. Coupons can potentially be printed out from the web tool, or may be printed out with the receipt at the point of sale along with other information such as the nutrition gap chart. Users may be able to choose from a number of recommended products, so that they are more comfortable with the dietary change, reducing the stress associated with the process of change.

The computer-implemented method embodying the present invention may be carried out at the demand of the user, or may occur periodically. Alternatively, the method may be performed each time the loyalty card associated with a particular diet is used in a transaction. According to the updated analysis, new product recommendations with new incentives are then given to the user. If the target nutritional composition is not achieved within a certain period, another set of recommended products are provided. This process is carried out until the gap value becomes zero.

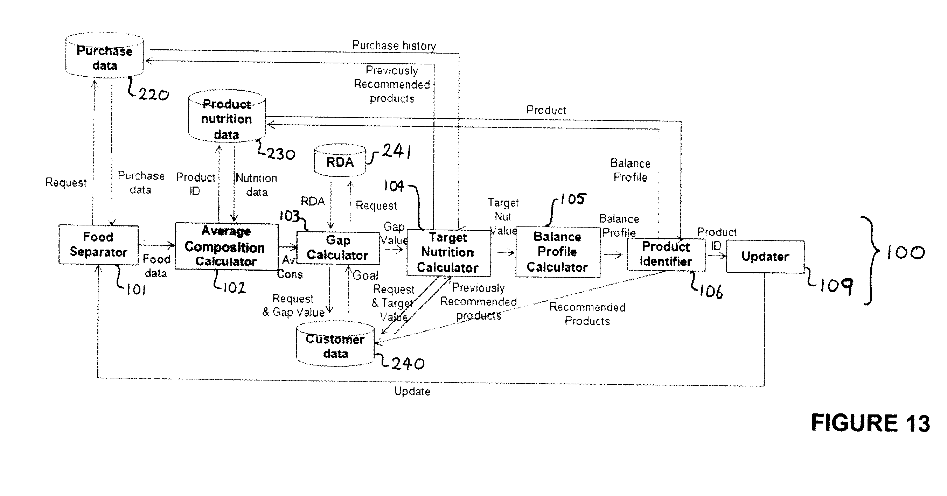

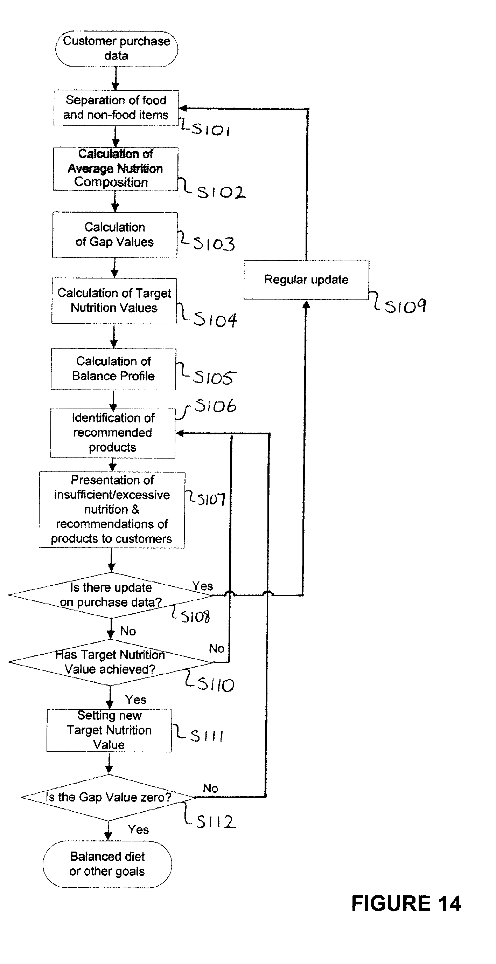

Figure 13 is a block diagram illustrating an apparatus embodying the present invention, and Figure 14 is a flowchart for the method performed by the apparatus of Figure 13. The food separator 101, average composition calculator 102, gap calculator 103, target nutrition calculator 104, balance profile calculator 105, product identifier 106, and updater 109 are all exemplary components of the personalised food recommendation mechanism 100 of Figure 2, and are an example of the apparatus 10 of Figure 1. The purchase data 220 is a database and is an example of the purchase list 20 in Figure 1. The product nutrition data 230 is a database and is an example of the nutritional information database 30 of Figure 1. The RDA 241 is a database. The customer data 240 is a database and is configured to store diet or consumer-specific data. Each of the databases may be incorporated into the personalised food recommendation mechanism 100, or may be provided remotely. The databases may be provided as distinct databases, as depicted, or may be combined into one or more joint databases. The arrows on Figure 13 represent data connections between the components, and between components and databases.

The food separator 101 is configured to carry out step S101 of Figure 14. The food separator 101 is an example of a purchase list access unit 11. The food separator 101 is operable to send a request for a purchase list as purchase data to the purchase data database 220. The purchase data database 220 is configured to receive the request from the food separator and respond with a purchase list as purchase data in accordance with the request. For example, the request may include a profile ID linked to a diet, whether it be a patient ID for a healthcare organisation, or a profile ID in a loyalty card system. The purchase list may include a list of historical purchases over a time period that is either predefined, or specified in the request. The purchase list may also include data relating to future purchases based on a current state of a shopping basket or a projection based on past purchases. The food separator 101 is configured to receive the purchase list as purchase data from the purchase data database 220 and to separate the food and other ingestible items from the non-food items. The food separator 101 is then configured to transfer the separated food and other ingestible item data as food data to the average composition calculator 102.

The average composition calculator 102 is an example of a component that combines the functions of the nutritional information database access unit 12 and the combination unit 13. The average composition calculator 102 is configured to perform the method step S102 of Figure 14. The average composition calculator 102 is configured to receive the food and other ingestible item data as food data from the food separator 101. The average composition calculator 102 is configured to send a request to the product nutrition data database 230 identifying each product ID in the food data, and to receive a nutritional profile as nutrition data in reply. The product nutrition data database 230 is configured to receive a request from the average composition calculator 102 identifying a product ID and to respond with a nutritional profile as nutrition data for that product ID. Based on factors including the time period covered by the food data, the amount of wastage (either predicted or actual) and the number of people represented by the profile ID for the diet, the average composition calculator 102 is configured to calculate average composition data, which may be expressed as an amount of each of a plurality of nutrients consumed per person in a given time period. The average composition calculator 102 is configured to transfer the calculated average composition data to the gap calculator 103.

The gap calculator 103 is an example of the nutrition gap chart generating unit 14. The gap calculator 103 is configured to perform step S103 of the flowchart of Figure 14. The gap calculator 103 is configured to receive the average composition data from the average composition calculator 102. The gap calculator is configured to send a request for RDA data from the RDA database. This may be performed periodically, on a per-usage basis, or alternatively the gap calculator may be pre-loaded with configurable RDA data. The RDA database 241 is configured to receive the request from the gap calculator 103 and to respond with RDA data for each of a plurality of nutrients. The gap calculator 103 is configured to send a request for the nutritional target composition for a diet to the customer data database 240. The gap calculator 103 is configured to receive the nutritional target composition for the diet from the customer data database 240 and to calculate, as a gap value on a per-nutrient basis, the difference between the nutritional target composition which the diet needs to contain to meet the target, and the current composition expressed by the average composition data. The gap calculator is then configured to generate a nutrition gap chart based on the calculated gap values. The nutrition gap chart may be, for example, a radar chart, a bar chart, or a pie chart. The gap calculator 103 is configured to send either the nutrition gap chart or the gap values to the customer data database 240 to be stored against the profile ID of the diet. The gap calculator 103 is also configured to send the nutrition gap chart to the target nutrition calculator 104.

The target nutrition calculator 104 and balance profile calculator 105 together form an example of the analysis unit 15. The target nutrition calculator 104 is configured to perform step S104 of the flowchart of Figure 14. The target nutrition calculator 104 is configured to receive the nutrition gap chart from the target nutrition calculator 104. The target nutrition calculator 104 is configured to calculate a component of a required balance profile for each of the plurality of nutrients in question. Optionally, as discussed above, the component may be calculated by dividing the gap value by a coefficient to take into account the target time period over which the diet is to be modified to achieve the target nutritional composition. The target nutrition calculator 104 is configured to send a request for the target time period for the diet in question to the customer data database 240, in response to which the customer data database is configured to send target time period data. Optionally, again as discussed above, the component of the required balance profile may take into account an engagement level. In order to obtain the engagement level, the target nutrition calculator 104 is configured to send a request for previously recommended product data to the customer data database 240. The customer data database 240 is configured to respond to a request for previously recommended product data with previously recommended product data for the diet in question.

Once the previously recommended product data has been received by the target nutrition calculator 104, the target nutrition calculator 104 is configured to forward the data to the purchase data database 220, which is configured to respond with data detailing which of the previously recommended products have been purchased and how many times, as recommended purchase history data. The target nutrition calculator is configured to obtain an engagement level value based on the recommended purchase history data and the previously recommended product data.

As discussed above in relation to the analysis unit 15, for each nutrient being considered, the target nutrition calculator 104 is configured to combine the gap value, the coefficient, and the engagement level value to obtain a component of the required balance profile. The components may be combined and expressed as a target nutrition value chart before being sent to the balance profile calculator 105, or may be sent as a list of values (target nutrition values).

The balance profile calculator 105 is configured to perform the step of S105 of the flowchart of Figure 14. The balance profile calculator 105 is configured to receive the target nutrition value chart or the target nutrition values from the target nutrition calculator 104. In accordance with the technique described above with reference to Figures 6 to 10, the balance profile calculator 105 is configured to calculate the required radar balance profile based on the received data, and to transfer the calculated required radar balance profile to the product identifier 106.

The product identifier 106 is an example of the ingestible product selection unit 16. The product identifier 106 is configured to perform step S106 of the flowchart of Figure 14. The product identifier 106 is configured to receive the required radar balance profile from the balance profile calculator 105. The product identifier 106 is configured to send the required radar balance profile, or data representing characteristics thereof such as the gradient or surrounding area values detailed above, to the product nutrition data database 230. The product nutrition data database 230 is configured to receive the required radar balance profile or data representing characteristics thereof from the product identifier 106 and to respond with the nutritional profile and product ID for one or more potential ingestible products. As detailed above in relation to the ingestible product selection unit with reference to Figures 11 and 12, the product identifier 106 is configured to function in the same way to select an ingestible product or products from the potential ingestible products. The product identifier 106 is then configured to transfer data identifying the selected ingestible product or products to the customer data database 240 for retrieval by a point of sale apparatus, a user via a web tool, or a retailer.

Optionally, the product identifier is configured to transfer a product ID of a selected product to the updater 109. The updater 109 is configured to receive the product ID and to send it to the food separator 101, for example, for investigating the potential effect of the selected product on the diet's composition.

The flowchart of Figure 14 represents a loop of control for updating and improving the diet's nutritional composition with respect to target nutritional composition on a continual basis. At step S107, the nutrition gap chart and recommendations based on the selected products, perhaps along with incentives, are presented to the user. For example, this may be performed via a GUI embodying the present invention.

At step S108, it is considered whether the purchase data has been updated. If there is new purchase data, for example a shopping basket has changed or a new transaction has been attributed to the relevant profile ID, then a regular update is performed by the updater 109 at step S109. The updater 109 is configured to control when the process of Figure 14, as performed by the personalised food recommendation mechanism 100, is performed, in particular, the updater S109 is configured to perform steps S108 to S112 of the flowchart of Figure 14. In the flowchart of Figure 14, it is event-triggered by new purchase data. For example, this could be the addition of an ingestible product to a shopping basket attributed to the user, and whilst the user is receiving and following recommendations from the product identifier 106 via the customer data database 240, the process of steps S101 to S107 repeats. That is to say, the customer data database 240 may be linked to a GUI embodying the present invention which embeds, includes, or is linked to, a website or GUI enabling a user to buy ingestible products or compose a list of products for a diet (such as a shopping list). If the answer to S108 is that, no, there is no update on purchase data, then the user may have finished choosing ingestible products and the flow proceeds to step S110.

At S110, if the target nutrition values have been achieved, or in the case of a target nutrition value chart, if the updated purchase data matches the chart, then it is not necessary to recommend any more products, and the flow proceeds to S111, at which a new set of target nutrition values, or a new target nutrition chart, is set for a forthcoming period of time. This may be done by the target nutrition calculator 104. If target nutrition value has not been achieved, the flow returns to step S106, and more products are recommended. These recommendations may not be received by a user until a next session or transaction, depending on the implementation. At step S112, the size of the gap values in terms of the difference between target nutritional composition and current nutritional composition is considered. If it is zero, then the balanced diet or other nutritional goal has been met. If not, the flow returns to step S106 and more recommendations are made.

Advantageously, in the embodiment described above, controllers have the strength of having a personal data for the person or people whose diet is being modified or designed with their scheme, for example a retailer has the advantage of having a customer's data with their loyalty scheme, and may be already utilising the data for other analysis. In the embodiment, purchase data available from the scheme is used to analyse a diet's nutritional composition.

Users can utilise the apparatus of the embodiment to check whether they or the people for whom they are designing or modifying a diet have a healthy eating lifestyle and to receive data identifying selected ingestible products as recommendations to improve the diet's nutritional composition and collect further loyalty points. It is up to the user to decide how much they personalise the service (i.e. more personal data, more personalisation).

The embodiment places an emphasis on reducing the stress level in the process of changing lifestyle, reducing the obstacles for consumers. It takes into account change management issues related to lifestyle change, which are often ignored. Instead of introducing dramatic change to their existing lifestyle, the embodiment provides food suggestions, which involve gradual changes in a diet's nutritional composition. In addition to balanced diet, other goals (such as reduced calories or reduced cholesterol) can be achieved with the same mechanism. For reduced calories, for example, food items with similar nutrition chart shape with less calories are recommended.

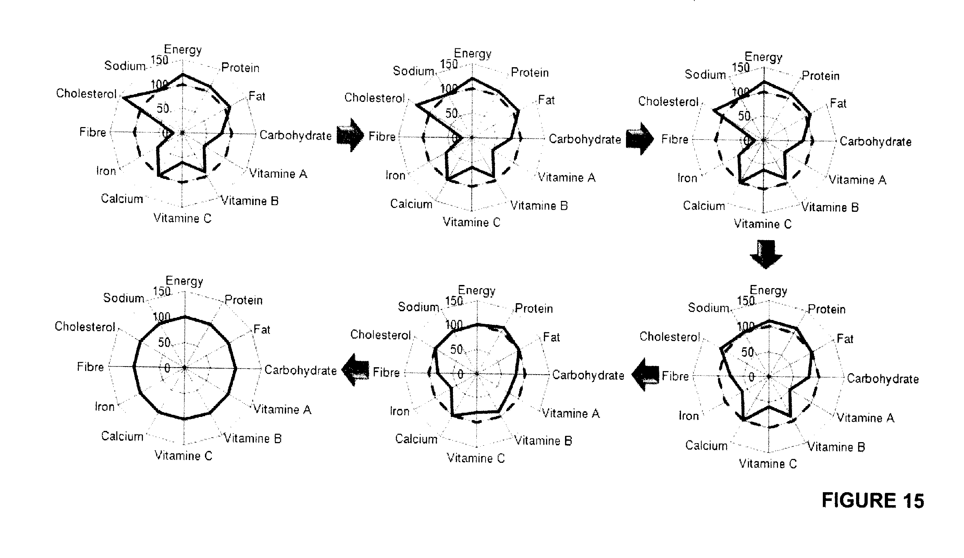

Figure 15 shows, in a radar chart format, the incremental steps by which the differences in the nutrition gap chart can be gradually reduced. The target nutritional composition is denoted by the dotted line, and the current nutritional composition by the solid line, accounting for a time lapse between each chart.

The embodiment above uses a graphical comparison for nutritional analysis because comparing nutritious numerical values themselves are too complicated and hence computationally expensive, and also to look at the whole balance of the nutrition of the alternative products, instead of some specific characteristics of the product. For example, it is not advisable to recommend to somebody, who has insufficient calcium intake, something which is high in calcium and also high in energy, if that person is trying to lose weight (which will be reflected in their target nutritional composition). In addition, those values can not be necessarily understood by the customers.

The results of nutrition analysis are also in a graphical form, making it easier for users and consumers to understand the implication of the results. From a diet's purchase data, current nutritional composition based on a purchase list of past purchases is accessed, and based on that nutritional intake, personalised recommendations are given to modify the diet to achieve a target nutritional composition and ultimately a health goal.

Since this mechanism uses data which have already been collected by controllers, or is made available to controllers via retailers, there is no need for the user to make a significant amount of effort. This can be used in line with loyalty points or some other incentive scheme, and therefore the user can also be motivated economically. It is completely up to the user whether to take the recommendations to achieve the diet's target nutritional composition and get more rewards or not. Controllers can empower users to manage their own health by providing a tool which is accessible on the web to facilitate continuous support for consumers to achieve their own goals.

This mechanism can be used in conjunction with other methods, such as food picture analysis. It should be noted that in the above detailed description the term ingestible product or item is used to encompass either one or a combination of ingestible products or items.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

The invention may be embodied as a computer program which, when executed by a processor, causes the computer to function as an apparatus embodying the present invention, as defined by the claims or otherwise.
Furthermore, the invention may be embodied as a computer program which, when executed by a processor, causes the computer to perform a method embodying the present invention, as defined by the claims or otherwise.

**Claims**

1. A computer-implemented method of selecting an ingestible product or combination of ingestible products for a diet, comprising the steps of:

   accessing a purchase list representing the diet's composition of ingestible products over a period of time in terms of product IDs;
   accessing a nutritional information database to obtain, for each product ID on the purchase list, a nutritional profile representing the amount of each of a plurality of nutrients in the product corresponding to the product ID;
   combining the purchase list with the nutritional profile for each product ID on the purchase list to obtain a nutritional purchase list, representing the diet's composition of ingestible products over the period of time in terms of the amount of each of the plurality of nutrients;
   generating a nutrition gap chart representing, for each of the plurality of nutrients, the difference between the nutritional purchase list and a nutritional composition target of the diet for the same period of time;
   performing a mathematical analysis on data represented by the nutrition gap chart to obtain, for one or more chosen nutrients from among the plurality of nutrients, a required balance profile in dependence upon the size of the difference or relative sizes of the differences; and
   selecting from an ingestible product database an ingestible product or combination of ingestible products having a nutritional balance profile representing the amount of the one or more chosen nutrients that matches the required balance profile to within a predetermined error margin.

2. The computer-implemented method according to claim 1, wherein the number of chosen nutrients is two or more.

3. The computer-implemented method according to claim 1 or 2, further comprising:

   displaying the nutrition gap chart as a radar chart.

4. The computer-implemented method according to claim 3, wherein
   the nutritional purchase list and the nutritional composition target are scaled with respect to the period of time for which the diet's composition of ingestible products is represented by the purchase list so that the nutrition gap chart represents an arbitrary time period.

5. The computer-implemented method according to any of claims 1 to 4, further comprising:

   setting the nutritional composition target of the diet based on diet-specific information.

6. The computer-implemented method according to any of claims 1 to 5, wherein
   two or more ingestible products or combinations of ingestible products are selected from the ingestible product database, each selected product or combination of products having a nutritional balance profile representing the amount of the one or more chosen nutrients that matches the required balance profile to within a predetermined error margin.

7. The computer-implemented method according to any of claims 1 to 6, wherein
   the nutrition gap chart is generated in response to a request, the purchase list includes a list of future purchases, and the method further comprises:

   responding to the request by displaying a graphical representation of the future nutrition gap chart.

8. The computer-implemented method according to any of claims 1 to 7, wherein
   the nutrition gap chart is generated in response to a request, the purchase list includes a list of past purchases, and the method further comprises:

responding to the request by displaying a graphical representation of the past nutrition gap chart.

9. The computer-implemented method according to claim 7 or 8, wherein the response to the request further comprises information identifying the selected ingestible product or combination of ingestible products of claim 1, or information identifying the two or more ingestible products or combinations of ingestible products of claim 6.

10. The computer-implemented method according to claim 9, wherein
each of the one or more ingestible products or combinations of ingestible products is graded according to an algorithm based on the amount of the one or more chosen nutrients present in the selected ingestible product or combination of ingestible products relative to the sizes of the differences for the chosen nutrients.

11. The computer-implemented method according to any of claims 1 to 10, wherein
the required balance profile is also dependent upon a time moderation factor representing a length of time by the end of which the diet's nutritional composition target is to be met.

12. The computer-implemented method according to any of claims 1 to 11, wherein
the purchase list for the diet is updated by a user or by a retailer linking a transaction identifying one or more product IDs to the diet.

13. The computer-implemented method according to any of claims 1 to 12, including:

generating a radar chart as the nutrition gap chart;
for each difference represented on the radar chart, generating an x-y coordinate corresponding to its position on the radar chart;
conducting a graphical analysis of the x-y coordinates to obtain a required radar balance profile as the required balance profile; and
selecting an ingestible product or combination of ingestible products by, for each of a number of potential ingestible products or combinations of ingestible products, generating a series of x-y coordinates corresponding to the position on a radar chart of the amount of the one or more chosen nutrients, conducting a graphical analysis of the series of x-y coordinates to obtain a potential product radar balance profile, comparing the required radar balance profile with the potential product radar balance profile, and assessing how closely they match.

14. A graphical user interface for presenting to a user information representing an ingestible product or combination of ingestible products, comprising:

a purchase list access module operable to access a purchase list representing a diet's composition of ingestible products over a period of time in terms of product IDs;
a nutritional information database access module operable to access a nutritional information database to obtain, for each product ID on the purchase list, a nutritional profile representing the amount of each of a plurality of nutrients in the product corresponding to the product ID;
a combination module operable to combine the purchase list with the nutritional profile for each product ID on the purchase list to obtain a nutritional purchase list, representing the diet's composition of ingestible products over the period of time in terms of the amount of each of the plurality of nutrients;
a nutrition gap chart display module operable to display a nutrition gap chart representing, for each of the plurality of nutrients, the difference between the nutritional purchase list and a nutritional composition target of the diet for the same period of time; and
a selected ingestible product display module operable to display information representing a selected ingestible product, the selected ingestible product being obtained by:

performing a mathematical analysis on data represented by the nutrition gap chart to obtain, for one or more chosen nutrients from among the plurality of nutrients, a required balance profile in dependence upon the size of the difference or relative sizes of the differences; and
selecting from an ingestible product database an ingestible product or combination of ingestible products having a nutritional balance profile representing the amount of the one or more chosen nutrients that matches the required balance profile to within a predetermined error margin.

15. An apparatus for selecting an ingestible product or combination of ingestible products for a diet, comprising:

a purchase list access unit configured to access a purchase list representing the diet's composition of ingestible products over a period of time in terms of product IDs;

a nutritional information database access unit configured to access a nutritional information database to obtain, for each product ID on the purchase list, a nutritional profile representing the amount of each of a plurality of nutrients in the product corresponding to the product ID;

a combination unit configured to combine the purchase list with the nutritional profile for each product ID on the purchase list to obtain a nutritional purchase list, representing the diet's composition of ingestible products over the period of time in terms of the amount of each of the plurality of nutrients;

a nutrition gap chart generating unit configured to generate a nutrition gap chart representing, for each of the plurality of nutrients, the difference between the nutritional purchase list and a nutritional composition target of the diet for the same period of time;

an analysis unit configured to perform a mathematical analysis on data represented by the nutrition gap chart to obtain, for one or more chosen nutrients from among the plurality of nutrients, a required balance profile in dependence upon the size of the difference or relative sizes of the differences;

an ingestible product selection unit configured to select from an ingestible product database an ingestible product or combination of ingestible products having a nutritional balance profile representing the amount of the one or more chosen nutrients that matches the required balance profile to within a predetermined error margin.

**Amended claims in accordance with Rule 137(2) EPC.**

**1.** A computer-implemented method of selecting an ingestible product or combination of ingestible products for a diet, the diet being a person or group of people's intake of ingestible products over a particular period of time, the method comprising the steps of:

accessing a purchase list representing the diet's composition of ingestible products over a period of time in terms of product IDs;

accessing a nutritional information database storing values of particular nutrients for each of a plurality of products to obtain, for each product ID on the purchase list, a nutritional profile representing the amount of each of a plurality of nutrients in the product corresponding to the product ID;

combining the purchase list with the nutritional profile for each product ID on the purchase list to obtain a nutritional purchase list, representing the diet's composition of ingestible products over the period of time in terms of the amount of each of the plurality of nutrients;

generating a radar chart as a nutrition gap chart representing, for each of the plurality of nutrients, the difference between the nutritional purchase list and a target nutritional composition of the diet for the same period of time, and for each difference represented on the radar chart, generating an x-y coordinate corresponding to its position on the radar chart;

conducting a graphical analysis of the x-y coordinates to obtain, for one or more chosen nutrients from among the plurality of nutrients, a required radar balance profile in dependence upon the size of the difference or relative sizes of the differences and the timescale within which the target nutritional composition is to be achieved, the required radar balance profile representing an amount of each of the one or more chosen nutrients, the amount being selected to remedy the difference or differences represented on the nutrition gap chart; and

selecting an ingestible product or combination of ingestible products by, for each of a number of potential ingestible products or combinations of ingestible products, generating a series of x-y coordinates corresponding to the position on a radar chart of the amount of the one or more chosen nutrients by accessing the nutritional information database, conducting a graphical analysis of the series of x-y coordinates to obtain a potential product radar balance profile, comparing the required radar balance profile with the potential product radar balance profile, and assessing how closely they match.

**2.** The computer-implemented method according to claim 1, wherein the number of chosen nutrients is two or more.

**3.** The computer-implemented method according to claim 1 or 2, further comprising:

displaying the nutrition gap chart as a radar chart.

**4.** The computer-implemented method according to claim 3, wherein
the nutritional purchase list and the nutritional composition target are scaled with respect to the period of time for which the diet's composition of ingestible products is represented by the purchase list so that the nutrition gap chart represents an arbitrary time period.

**5.** The computer-implemented method according to any of claims 1 to 4, further comprising:

setting the nutritional composition target of the diet based on diet-specific information.

**6.** The computer-implemented method according to any of claims 1 to 5, wherein
two or more ingestible products or combinations of ingestible products are selected from the ingestible product database, each selected product or combination of products having a nutritional balance profile representing the amount of the one or more chosen nutrients that matches the required balance profile to within a predetermined error margin.

**7.** The computer-implemented method according to any of claims 1 to 6, wherein
the nutrition gap chart is generated in response to a request, the purchase list includes a list of future purchases, and the method further comprises:

responding to the request by displaying a graphical representation of the future nutrition gap chart.

**8.** The computer-implemented method according to any of claims 1 to 7, wherein
the nutrition gap chart is generated in response to a request, the purchase list includes a list of past purchases, and the method further comprises:

responding to the request by displaying a graphical representation of the past nutrition gap chart.

**9.** The computer-implemented method according to claim 7 or 8, wherein the response to the request further comprises information identifying the selected ingestible product or combination of ingestible products of claim 1, or information identifying the two or more ingestible products or combinations of ingestible products of claim 6.

**10.** The computer-implemented method according to claim 9, wherein
each of the one or more ingestible products or combinations of ingestible products is graded according to an algorithm based on the amount of the one or more chosen nutrients present in the selected ingestible product or combination of ingestible products relative to the sizes of the differences for the chosen nutrients.

**11.** The computer-implemented method according to any of claims 1 to 10, wherein
the required balance profile is also dependent upon a time moderation factor representing a length of time by the end of which the diet's nutritional composition target is to be met.

**12.** The computer-implemented method according to any of claims 1 to 11, wherein
the purchase list for the diet is updated by a user or by a retailer linking a transaction identifying one or more product IDs to the diet.

**13.** An apparatus for selecting an ingestible product or combination of ingestible products for a diet, the diet being a person or group of people's intake of ingestible products over a particular period of time, the apparatus comprising:

a purchase list access unit configured to access a purchase list representing the diet's composition of ingestible products over a period of time in terms of product IDs;
a nutritional information database access unit configured to access a nutritional information database storing values of particular nutrients for each of a plurality of products to obtain, for each product ID on the purchase list, a nutritional profile representing the amount of each of a plurality of nutrients in the product corresponding to the product ID;
a combination unit configured to combine the purchase list with the nutritional profile for each product ID on the purchase list to obtain a nutritional purchase list, representing the diet's composition of ingestible products over the period of time in terms of the amount of each of the plurality of nutrients;
a nutrition gap chart generating unit configured to generate a radar chart as a nutrition gap chart representing, for each of the plurality of nutrients, the difference between the nutritional purchase list and a target nutritional composition of the diet for the same period of time, and for each difference represented on the radar chart, to generate an x-y coordinate corresponding to its position on the radar chart;
an analysis unit configured to conduct a graphical analysis of the x-y coordinates to obtain, for one or more chosen nutrients from among the plurality of nutrients, a required radar balance profile in dependence upon the size of the difference or relative sizes of the differences and the timescale within which the target nutritional composition is to be achieved, the required radar balance profile representing an amount of each of the one or more chosen nutrients, the amount being selected to remedy the difference or differences represented on the nutrition gap chart;

an ingestible product selection unit configured to select an ingestible product or combination of ingestible products by, for each of a number of potential ingestible products or combinations of ingestible products, generating a series of x-y coordinates corresponding to the position on a radar chart of the amount of the one or more chosen nutrients by accessing the nutritional information database, conducting a graphical analysis of the series of x-y coordinates to obtain a potential product radar balance profile, comparing the required radar balance profile with the potential product radar balance profile, and assessing how closely they match.

| Purchase List | Nutritional information database |
| --- | --- |
| 20 | 30 |

| Purchase List access unit | Nutritional information database access unit | Combination unit | Nutrition gap chart generating unit | Analysis unit | Ingestible product selection unit |
| --- | --- | --- | --- | --- | --- |
| 11 | 12 | 13 | 14 | 15 | 16 |

10

**FIGURE 1**

**FIGURE 2**

Energy

140

Sodium 120 Protein

100

80 Fat

Cholesterol 60

40

20

Fibre 0 Carbohydrate

Iron Vitamine A

Calcium Vitamine B

Vitamine C

EP 2 518 649 A1

**FIGURE 3**

(a)

(b)

FIGURE 4

FIGURE 5

FIGURE 6

FIGURE 7

| n | Nutrition | Gap Value (Gn) | Using Trigonometric functions (Xn, Yn) | Using Vector calculus | |
|---|---|---|---|---|---|
| | | | | Gradient An between Vn and Vn-1 | Constant Bn |
| 1 | Energy | 0 | (V1sin(30*(N-1)), V1cos(30*(N-1)) | (X2-X1)/(Y2-Y1) | (Y1-A1X1) |
| 2 | Protein | 0 | (V2sin(30*(N-1)), V2cos(30*(N-1)) | (X3-X2)/(Y3-Y2) | (Y2-A2X2) |
| 3 | Fat | 0 | (V3sin(30*(N-1)), V3cos(30*(N-1)) | (X4-X3)/(Y4-Y3) | (Y3-A3X3) |
| 4 | Carbohydrate | 20 | (V4sin(30*(N-1)), V4cos(30*(N-1)) | (X5-X4)/(Y5-Y4) | (Y4-A4X4) |
| 5 | Vitamin A | 50 | (V5sin(30*(N-1)), V5cos(30*(N-1)) | (X6-X5)/(Y6-Y5) | (Y5-A5X5) |
| 6 | Vitamin B | 10 | (V6sin(30*(N-1)), V6cos(30*(N-1)) | (X7-X6)/(Y7-Y6) | (Y6-A6X6) |
| 7 | Vitamin C | 40 | (V7sin(30*(N-1)), V7cos(30*(N-1)) | (X8-X7)/(Y8-Y7) | (Y7-A7X7) |
| 8 | Calcium | 0 | (V8sin(30*(N-1)), V8cos(30*(N-1)) | (X9-X8)/(Y9-Y8) | (Y8-A8X8) |
| 9 | Iron | 40 | (V9sin(30*(N-1)), V9cos(30*(N-1)) | (X10-X9)/(Y10-Y9) | (Y9-A9X9) |
| 10 | Fibre | 80 | (V10sin(30*(N-1)), V10cos(30*(N-1)) | (X11-X10)/(Y11-Y10) | (Y10-A10X10) |
| 11 | Cholesterol | 0 | (V11sin(30*(N-1)), V11cos(30*(N-1)) | (X12-X11)/(Y12-Y11) | (Y11-A11X11) |
| 12 | Sodium | 0 | (V12sin(30*(N-1)), V12cos(30*(N-1)) | (X1-X12)/(Y1-Y12) | (Y12-A12X12) |

FIGURE 8

_If X4 < X5_

$\triangle OV5V4 = \triangle OV5Z - \triangle V5ZV4$

_If X4 > X5_

$\triangle OV5V4 = \triangle OV5Z + \triangle V5V4Z$

_If X5 < X6_

$\triangle OV6V5 = \triangle OV6Z2 - \square V5V6Z2Z1 - \triangle OV5Z1$

_If X5 > X6_

$\triangle OV6V5 = \square V6V5Z2Z1 + \triangle OV6Z1 - \triangle OV5Z2$

**FIGURE 9**

28

*For both X6 < X7 and X6 > X7*

$$\triangle OV7V6 = \square OV7V6Z - \triangle OV6Z$$

**FIGURE 10**

(a)

Broccoli (10 points)

(b)   Baked beans (15 points)

# FIGURE 11

(a)

(b)

Beef (10 points)

**FIGURE 12**

**FIGURE 13**

**FIGURE 14**

Customer purchase data

Separation of food and non-food items — S101

Calculation of Average Nutrition Composition — S102

Calculation of Gap Values — S103

Calculation of Target Nutrition Values — S104

Calculation of Balance Profile — S105

Identification of recommended products — S106

Presentation of insufficient/excessive nutrition & recommendations of products to customers — S107

Regular update — S109

Is there update on purchase data? — S108 — Yes / No

Has Target Nutrition Value achieved? — S110 — No / Yes

Setting new Target Nutrition Value — S111

Is the Gap Value zero? — S112 — No / Yes

Balanced diet or other goals

**FIGURE 15**

Europäisches
Patentamt

European
Patent Office

Office européen
des brevets

# EUROPEAN SEARCH REPORT

Application Number

EP 11 16 3784

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/086374 A1 (AITKEN STUART [US] ET AL) 10 April 2008 (2008-04-10) <br> * paragraph [0007] * <br> * paragraph [0032] * <br> * paragraph [0036] - paragraph [0037] * <br> * paragraph [0042] - paragraph [0045] * <br> * paragraph [0055] - paragraph [0056] * <br> * claim 1 * <br> ----- | 1-15 | INV. <br> G06F19/00 |
| X | US 2009/099873 A1 (KURPLE KARL VINCENT [US]) 16 April 2009 (2009-04-16) <br> * paragraph [0005] - paragraph [0006] * <br> * paragraph [0076] * <br> * paragraph [0087] * <br> * paragraph [0090] * <br> * paragraph [0093] * <br> * paragraph [0096] - paragraph [0097] * <br> * claim 1; figure 5 * <br> ----- | 1-15 | |
| X | GB 2 439 670 A (SETUP VENTURES LICENSING LTD [GB]) 2 January 2008 (2008-01-02) <br> * page 1, line 26 - page 2, line 14 * <br> * page 9, line 30 - page 10, line 25 * <br> * page 22, line 12 - line 21 * <br> ----- | 1-15 | TECHNICAL FIELDS SEARCHED (IPC) <br><br> G06F |
| A | US 7 361 143 B2 (KIRCHHOFF DAVID [US] ET AL) 22 April 2008 (2008-04-22) <br> * column 12, line 44 - line 52 * <br> * column 13, line 20 - line 27 * <br> * column 15, line 3 - line 59 * <br> ----- | 1-15 | |

The present search report has been drawn up for all claims

| Place of search | Date of completion of the search | Examiner |
|---|---|---|
| Munich | 18 July 2011 | Sisk, Aisling |

EPO FORM 1503 03.82 (P04C01)

**ANNEX TO THE EUROPEAN SEARCH REPORT
ON EUROPEAN PATENT APPLICATION NO.**

EP 11 16 3784

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2011

| Patent document cited in search report | | Publication date | Patent family member(s) | | Publication date |
|---|---|---|---|---|---|
| US 2008086374 | A1 | 10-04-2008 | NONE | | |
| US 2009099873 | A1 | 16-04-2009 | NONE | | |
| GB 2439670 | A | 02-01-2008 | NONE | | |
| US 7361143 | B2 | 22-04-2008 | US 2003187683 | A1 | 02-10-2003 |
| | | | US 2004210459 | A1 | 21-10-2004 |
| | | | US 2004210456 | A1 | 21-10-2004 |
| | | | US 2004171925 | A1 | 02-09-2004 |
| | | | US 2004204955 | A1 | 14-10-2004 |

EPO FORM P0459

For more details about this annex : see Official Journal of the European Patent Office, No. 12/82